# EUROPEAN PATENT SPECIFICATION

(11) **EP 1 644 080 B1**
(45) Date of publication and mention of the grant of the patent: **04.05.2011**
(21) Application number: 04776701.7
(22) Date of filing: 16.06.2004
(51) Int. Cl.: A61N 1/375, A61N 1/05

(54) **MEDICAL LEAD ADAPTOR**
ADAPTER FÜR MEDIZINISCHE ZULEITUNG
ADAPTATEUR DE FIL MEDICAL

(30) Priority: 16.07.2003 US 620710
(43) Date of publication of application: 12.04.2006
(73) Proprietor: Medtronic, Inc., Minneapolis, MN 55432-5604 (US)
(72) Inventor: HOLLEMAN, Timothy, J., Ham lake, MN 55303 (US); RIES, Andrew, J., Lino Lakes, MN 55014 (US)
(74) Representative: Hughes, Andrea Michelle
(86) International application number: PCT/US2004/019352
(87) International publication number: WO 2005/009534

(56) References cited:
- WO-A-00/11762
- US-A- 4 628 934
- US-A- 5 514 161
- US-A- 5 766 042
- US-B1- 6 466 824

## Description

The present invention relates generally to medical systems with electrical leads and more particularly to means for connecting more than one medical lead terminal, each having a high-voltage contact, to a single connector port of an implantable medical device (IMD).

### BACKGROUND OF THE INVENTION

In the field of therapeutic electrical stimulation, it is often desirable to provide electrical connection of more than one lead to a single connection port of a medical device. In particular, in the field of cardiac pacing, it is sometimes necessary to provide electrical connection of two leads to a single connection port of an IMD, *e.g*. a cardiac pacemaker or implantable cardioverter defibrillator, such that stimulation pulses may be delivered to more than one cardiac site or across a desired vector.

Medical lead connectors have been standardized in the industry. For example IS-1 pacing/sensing connectors are available in unipolar or bipolar configurations, including one or two electrical contacts, respectively, for making connection between a unipolar pace/sense electrode or a bipolar pace/sense electrode pair and an IMD; DF-1 connectors are available for making a high-voltage connection between an IMD and a defibrillation electrode via a single a single electrical contact. Other contemplated standards define connectors for making both high-voltage and low-voltage connections between an IMD and multiple electrodes; one such standard defines a connector including two high voltage contacts and two low voltage contacts, effectively combining two unipolar DF-1 connectors and a bipolar IS-1 connector into a single connector in order to provide electrical coupling to two high-voltage electrodes and a bipolar pace/sense electrode pair carried on a single, quadripolar lead for pacing and defibrillating the heart.

Clinical experience has shown that, in some patients, an acceptable defibrillation threshold cannot be reached using a single lead. In these patients, it becomes necessary to implant more than one lead in order to create an effective vector for the delivery of defibrillation energy. It is therefore desirable to provide means allowing connection of two connectors, each including a high voltage contact, to a single connector port of an IMD.

WO 00/11762 A discloses a lead having an auxiliary part, thus providing means for connecting two leads to one single connector port of an IMD.

### BRIEF DESCRIPTION OF THE DRAWINGS

Embodiments of the present invention will be more readily understood from the following detailed description when considered in conjunction with the drawings, in which like reference numerals indicate identical structures throughout the several views, and wherein:
Figure 1 is plan view of a medical system including a lead adaptor described by way of background only;
Figure 2 is a sectional view of the adaptor of Figure 1;
Figure 3 is a sectional view of the connector terminal portion of an alternative embodiment of the adaptor of Figure 2;
Figure 4 is a sectional view of an alternative embodiment of the adaptor of Figure 2;
Figure 5 is a plan view of a trifurcated adaptor;
Figure 6 is a sectional view of the adaptor of Figure 5;
Figure 7 is plan view of a medical system according to an embodiment of the present invention; and
Figure 8 is a plan view of a medical system according to another alternate embodiment of the present invention.

The drawings are not necessarily to scale.

### DETAILED DESCRIPTION OF THE INVENTION

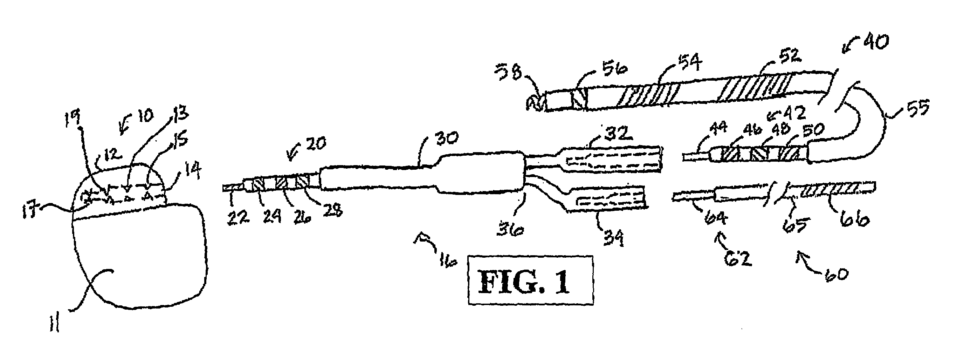

Figure 1 is plan view of a medical system, not forming part of the invention, including a lead adaptor 16.

Figure 1 illustrates the medical system including the adaptor 16 provided to couple a first lead 40 and a second lead 60 to an IMD 10. As illustrated in Figure 1, first lead 40 includes a tip electrode 58 and ring electrode 56, which provide a low-voltage therapy, for example pacing and sensing, and two coil electrodes 52 and 54, which provide high-voltage therapy, for example defibrillation. Each electrode 52, 54, 56, and 58 is electrically connected to a corresponding connector element 50, 48,46, and 44, respectively, located on a lead connector terminal 42 via electrically isolated conductors carried by a lead body 55. Figure 1 further illustrates second lead 60 including a coil electrode 66 for high voltage therapy; electrode 66 is electrically connected to a connector pin 64 included in a lead connector terminal 62 via an insulated conductor carried by a lead body 65. Here if electrodes 54 and 52 of first lead 40, when implanted in a patient, cannot be positioned to provide an acceptable defibrillation threshold, electrode 66 of second lead 60 is implanted in a position where it may be employed to improve the threshold, and both first lead 40 and second lead 60 are coupled to a single connector port 14 of IMD 10 via adaptor 16.

Adaptor 16 includes a connector terminal 20 adapted to engage within connector port 14 of a connector header 12 of IMD 10, and, as illustrated in Figure 1, connector terminal 20 includes three connector rings 24, 26 and 28 and a connector pin 22. Connector header 12 is shown attached to a hermetically sealed enclosure or can 11 that contains a battery and electronic circuitry and other components. Can 11 may further serve as a high voltage electrode in conjunction with lead electrodes 54, 52, 66. Port 14, configured to receive either first lead connector terminal 42 or adaptor connector terminal 20, includes high-voltage connectors 13 and 15 of any of the known types that are electrically connected to the electronic circuitry through feedthrough pins of feedthroughs (not shown) mounted to extend through can 11. Connectors 13 and 15 are dimensioned in diameter and are spaced apart in port 14 to receive and make electrical and mechanical connection with connector rings 26 and 28, respectively, of adaptor connector terminal 20, or with connector elements 48 and 50 of lead connector terminal 42. Such electrical and mechanical connection is effected either through the tightening of setscrews (not shown) as disclosed in U.S. Patent Nos. 4,142,532 and 4,182,345, for example, or an action of inwardly extending force beams (not shown) as disclosed in U.S. Patent Nos. 5,070,605 and 5,766,042, for example. Additional connectors 17 and 19 included in port 14 make mechanical and electrical contact with connector pin 22 and connector ring 24 of adaptor connector terminal 20 or with connector elements 44 and 46 of lead connector terminal 42. According to one embodiment, connector pin 22 and connector ring 24 are adapted for low-voltage coupling in port 14 while connector rings 26 and 28 are adapted for high-voltage coupling in port 14. According to alternate embodiments any two of connector rings 24, 26, 28 and connector pin 22 may be eliminated and a remaining two be adapted for high-voltage coupling in port 14.

As illustrated in Figure 1, adaptor 16 further includes a first receptacle port 32 and a second receptacle port 34 extending from a bifurcation 36 of an insulating body 30, which carries and electrically isolates conductors (not shown) extending from connector pin 22 and connector rings 24, 26, and 28 of adaptor connector terminal 20 to corresponding contacts included in receptacle ports 32 and 34. First port 32 and second port 34 are adapted to engage first lead connector 42 and second lead connector 62, respectively, providing electrical coupling for at least the high voltage electrodes of each lead. In some embodiments, second lead connector 62 conforms to the DF-1 connector standard.

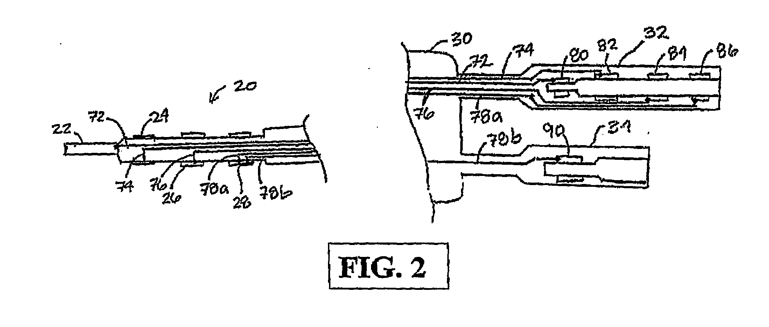

Figure 2 is a sectional view of adaptor 16 wherein receptacle port 32 is shown to include low-voltage contacts 80 and 82 and high-voltage contacts 84 and 86 for providing electrical connection to lead connector elements 44, 46, 48, and 50 respectively (Figure 1); contact 80 is coupled to connector pin 22 and via a conductor 72 and each of contacts 82, 84, and 86 are electrically coupled to connector rings 24, 26, and 28, respectively via conductors 74, 76, and 78a. Conductors 72, 74, 76 and 78a, extending through body 30 to adaptor connector terminal 20, are electrically isolated from one another.

Figure 2 further illustrates receptacle port 34 including a high-voltage contact 90 for providing electrical connection to lead connector pin 64 of second lead 60. Contact 90 is electrically coupled to a conductor 78b, which is further coupled to connector ring 28 of connector terminal 20. Thus, according to some embodiments, adaptor 16 performs as a signal splitter, providing common electrical connection for two high-voltage contacts 86 and 90 adapted to engage with high-voltage connector elements 50 and 64 located on separate leads 40 and 60, thereby enabling electrode 66 of second lead 60 to augment high-voltage therapy delivery of first lead 40.

Adaptor body 30 and external portions of receptacle 32 and 34 and connector assembly 20 may be formed from a biocompatible, insulating material known for use in manufacturing medical electrical leads. Appropriate materials include, but are not limited to, a polyurethane or silicone rubber. Conductors 72, 74, 76 and 78a may be provided as straight wire conductors, cabled conductors, coiled conductors or other types of conductors known for use in medical electrical leads or adaptors. Conductors 72, 74, 76, and 78a may extend through individual lumens formed within adaptor body 30 or may be individually insulated by a polymeric coating or tubing, such as PTFE or ETFE, and extend through a common central lumen formed within adaptor body 30.

While conductor 78b is shown coupled to contact 90 at a first end and connector ring 28 at a second end, the second end may alternatively be coupled to a node any where along conductor 78a. In this way, the high-voltage contact 84 of receptacle 32 and high-voltage contact 90 of receptacle 34 are both electrically coupled to the high-voltage connector ring 28 of adaptor connector terminal 20. In alternative embodiments, conductor 78b may be coupled to connector ring 26 or anywhere along conductor 76 such that a signal from connector ring 26 is split to high-voltage contact 84 of receptacle 32 via conductor 76 and to high-voltage contact 90 via conductor 78b.

Thus, an additional lead 60 including a high-voltage coil electrode 66 may be coupled to IMD 10 having a single connector port 14. Adaptor 16 allows placement of an additional, high-voltage lead in operative relation to the heart, without requiring a different IMD having an additional connector port, in order to achieve improved cardioversion or defibrillation thresholds when thresholds achieved with a single lead are unacceptably high.

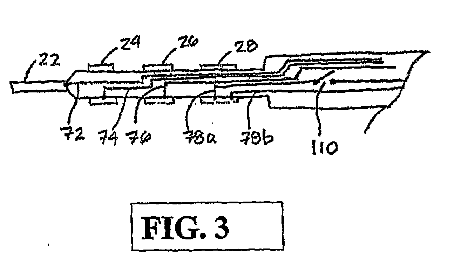

It is recognized that in some embodiments a first lead may have only one high-voltage electrode, for example electrode 54 of lead 40 illustrated in Figure 1; in this case, when a vector created between electrode 54 and can 11, acting as an electrode, does not provide an acceptably low defibrillation threshold a second lead including a high-voltage electrode, for example electrode 66 of lead 60, is coupled with the first lead to IMD 10 via adaptor 16 such that electrode 54 of the first lead is electrically coupled via contact 84 of first port 32, and electrode 66 of second lead 60 is coupled via contact 90 of second port 34 (Figure 2). According to these embodiments contact 86 and conductor 78a are not necessary elements of adaptor 16. Additional alternate embodiments employ a switch as illustrated in Figure 3.

Figure 3 is a sectional view of the connector terminal portion of an alternative embodiment of the adaptor of Figure 2 wherein a switch 110 is provided to allow electrical decoupling of a receptacle contact. When defibrillation thresholds achieved using coil electrodes on a first lead, for example electrodes 54 and 52 of lead 40 shown in Figure 1, are unacceptably high such that placement of a second high-voltage lead, for example lead 60, is required, it may be desirable to provide a high-voltage signal to the second lead without providing the same high-voltage signal to a coil electrode on the first lead. As such, switch 110 is provided between connector ring 28 and conductor 78a or anywhere along conductor 78a, which is coupled to contact 86 as shown previously in Figure 2. Switch 110 may be an electrically-, mechanically-, or magnetically-actuated switch. With switch 110 normally closed, a signal delivered to connector ring 28 is split between contact 86 of receptacle 32 and contact 90 of receptacle 34 via conductors 78a and 78b, respectively. When switch 110 is opened, contact 86 is electrically disconnected from connector ring 28 such that a signal delivered to connector ring 28 is conducted only to contact 90 via conductor 78b.

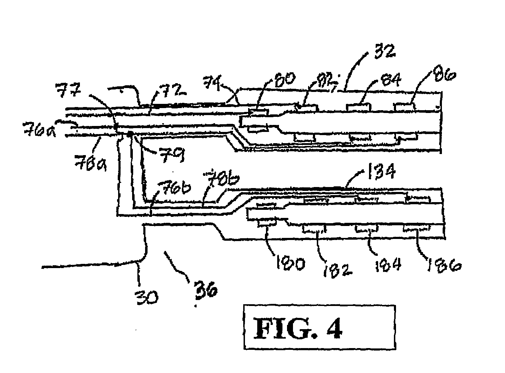

Figure 4 is a sectional view of an alternative embodiment of the adaptor of Figure 2 wherein a second port 134 is adapted to engage another multi-polar lead including a connector terminal similar to that of first lead 40 illustrated in Figure 1. According to this embodiment, signals delivered to two high-voltage connector rings are split to contacts engaging with connector elements of two separate leads. As such, receptacle 134 includes four contacts 180, 182, 184, and 186. Conductor 78b is shown in Figure 4 to be coupled at one end to high-voltage contact 186 and at the other end to conductor 78a at node 79, which may be located anywhere along the length of conductor 78a. Conductor 78a is further coupled to connector ring 28 of adaptor connector terminal 20 (Figures 2 and 3). Conductor 76b is coupled at one end to high-voltage contact 184 and at the other end to conductor 76a at node 77, which may be located anywhere along the length of conductor 76a. Conductor 76a is further coupled to connector ring 26 on adaptor connector terminal 20 (Figures 2 and 3). Thus, both high-voltage connector rings 26 and 28 of adaptor connector terminal 20 are coupled to high-voltage contacts in receptacles 32 and 134 allowing connection of two leads, each having two high-voltage coil electrodes, to a single connection port of an IMD. Signals delivered to connector ring 26 are split between contacts 84 and 184 and signals delivered to connector ring 28 are split between contacts 86 and 186. In alternative embodiments, conductors 76b and 78b may be coupled directly to connector rings 26 and 28 of connector terminal 20 rather than to nodes 77 and 79 as shown in Figure 4. Contacts 180 and 182 may be left inactive as shown in Figure 4, *i.e.,* having no electrical connection to conductors extending to adaptor connector terminal 20. In other embodiments, additional conductors may be provided for coupling additional contacts that may be present in receptacle 134 to connector elements included in connector terminal 20.

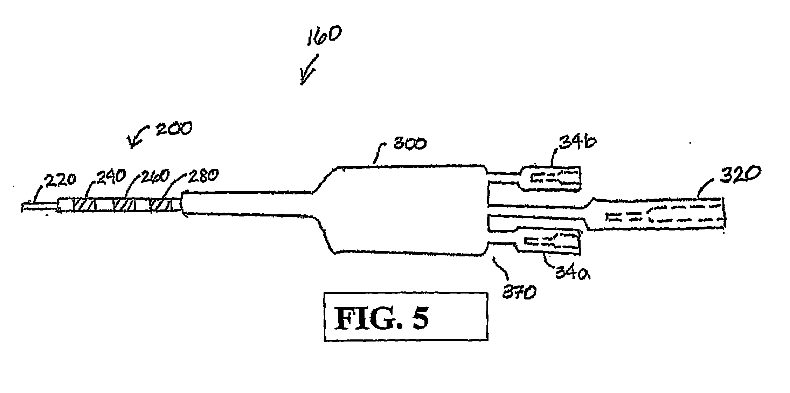

Figure 5 is a plan view of yet another embodiment of an adaptor 160 provided for splitting signals delivered to two high-voltage contacts engaging connector elements located on a first lead, for example connector elements 48 and 50 of lead 40 illustrated in Figure 1, and two additional high-voltage contacts located in two separate ports 34a and 34b for engaging connector elements of two separate leads, for example 64 of lead 60. Figure 5 illustrates a connector terminal 20 of adaptor 160 extending from an adaptor body 300 and including a connector pin 220 and connector rings 240, 260 and 280. Three receptacle ports 32, 34a and 34b extend from a trifurcation 370 at the opposite end of adaptor body 300.

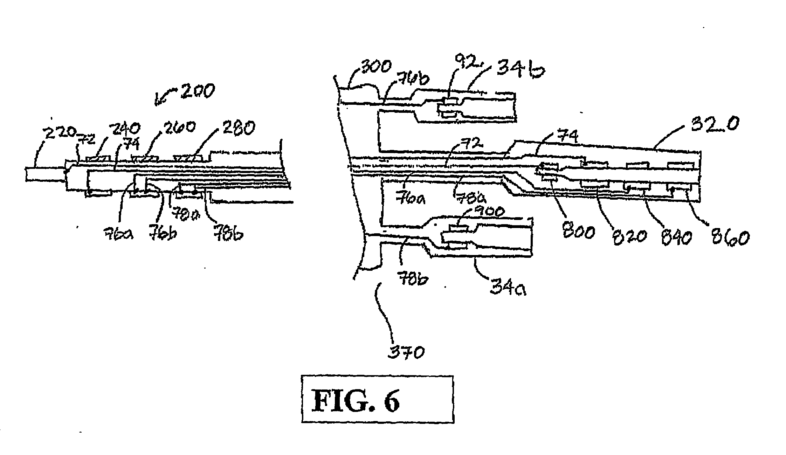

As shown in more detail in the sectional view of Figure 6, receptacle port 320 includes include low-voltage contacts 800 and 820 and high-voltage contacts 840 and 860 for providing electrical connection to lead connector elements 44, 46, 48, and 50 respectively (Figure 1); contact 800 is coupled to connector pin 220 and via a conductor 72 and each of contacts 820, 840, and 860 are electrically coupled to connector rings 240, 260, and 280, respectively via conductors 74, 76a, and 78a. Conductors 72, 74, 76a and 78a, extending through body 30 to adaptor connector terminal 20, are electrically isolated from one another.

Figure 6 further illustrates receptacle ports 34a and 34b each including a single, high-voltage contact 900 and 92, respectively. Ports 34a and 34b may be adapted to receive standard DF-1 lead connectors. Conductor 78b is coupled between contact 900 of receptacle port 34a and connector ring 280 of adaptor connector terminal 200 such that signals delivered to connector ring 280 are split between contact 860 of receptacle port 320 and contact 900 via conductors 78a and 78b, respectively. Conductor 76b is coupled between contact 920 of receptacle port 34b and connector ring 260 of adaptor connector terminal 200. Signals delivered to connector ring 260 are split between contact 840 of receptacle port 320 and contact 92 via conductors 76a and 76b, respectively. Adaptor 160 of Figures 5 and 6 thus allows connection of two additional high-voltage leads to an IMD having a single connection port, for example IMD 10 shown in Figure 1.

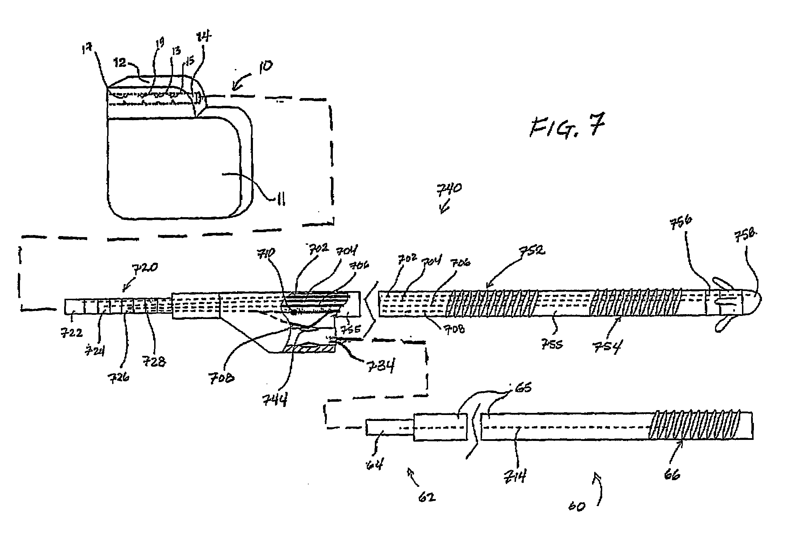

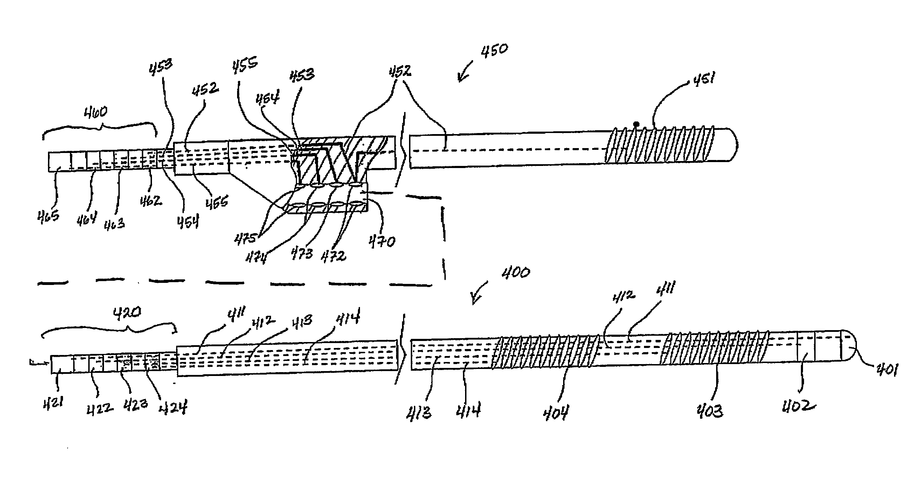

Figure 7 is plan view of a medical system according to an embodiment of the present invention wherein means for connecting two medical electrical leads to single port 14 of IMD 10 includes an auxiliary port 734 built into a first lead 740. Figure 7 illustrates first medical electrical lead 740 including a tip electrode 758 and a ring electrode 756, which provide low-voltage therapy, for example pacing and sensing, and two coil electrodes 752 and 754, which provide high voltage therapy, for example defibrillation. Each electrode 752, 754, 756, and 758 is electrically coupled to a corresponding connector element 728, 726, 724, and 722, respectively, located on a lead connector terminal 720 via electrically isolated conductors 708, 706, 704, and 702, respectively, carried by a lead body 755. Conductors 708, 706, 704, and 702 may be straight wire conductors, cabled conductors, coiled conductors or other types of conductors known for use in medical electrical leads or adaptors and may extend through individual lumens formed within lead body 755 or may be individually insulated by a polymeric coating or tubing, such as PTFE or ETFE, and extend through a common central lumen formed within lead body 755. Connector terminal 720 is adapted to engage within connector port 14 of connector header 12 of IMD 10, which is described herein in conjunction with Figure 1.

Figure 7 further illustrates auxiliary port 734 of first lead 740 including a connector contact 744 coupled to connector element 728 via conductor 708. Connector contact 744 may be a spring contact or setscrew contact, both types being well known to those skilled in the art. According to the present invention auxiliary port 734 is adapted to engage connector terminal 62 of second lead 60 in order to electrically couple electrode 66 of second lead 60 to connector element 728 of connector terminal 720, via contact between connector contact 744 and connector pin 64, which is coupled to electrode 66 by conductor 714. As previously described in conjunction with Figure 1, electrode 66 of second lead 60 may be implanted in a position to create a more effective defibrillation vector with an electrode or electrodes of a first lead, which in this case are electrodes 752 and 754 of first lead 740, in order to reduce a defibrillation threshold.

Figure 7 also illustrates a switch 710 not forming part of the invention, which is adapted to reversibly decouple electrode 752 from connector element 728 when only electrode 66 of second lead, coupled via port 734, and electrode 754 of first lead provide a desired defibrillation threshold. With switch 110 normally closed, a defibrillation pulse delivered from IMD 10, via connector element 728 of connector terminal 720, is split between electrode 752 of first lead and electrode 66 of second lead, while, when switch is open, the defibrillation pulse is sent only to electrode 66. Switch 710 may be an electrically-, mechanically-, or magnetically-actuated switch.

Although Figure 7 illustrates conductor 708 coupling connector contact 744 and electrode 752 to connector element 728 this does not form part of the invention. According to the present invention, a first lead does not include electrode 752 so that conductor 708 only couples contact 744 to connector element 728. Furthermore, in additional embodiments, first lead 740 does not include electrode 756 and associated conductor 704 and connector element 724, in which case electrode 758 operates in a unipolar pace/sense mode or in an integrated bipolar pace/sense mode in conjunction with electrode 754, both modes being well known to those skilled in the art. In yet another embodiment first lead 740 does not include coil electrodes 752 and 754, in which case one of conductors 706 and 708 is not included and the other only couples contact 744 to one of connector elements 726 and 728, providing means to augment a pace/sense system with coil electrode 66 via engagement of connector terminal 62 in port 734; electrode 66 forming a defibrillation vector with either can 11 or a coil electrode on yet another lead (not shown) included in the system.

Figure 8 is a plan view of a medical system according to yet another alternate embodiment of the present invention. Figure 8 illustrates a first lead 450 including a high-voltage electrode 451, a connector terminal 460 and an auxiliary port 470; electrode 451 is shown coupled, via a first insulated conductor 452, to both a connector element 462 of connector terminal 460 and to a connector contact 472 of auxiliary port 470. Connector terminal 460 is adapted to engage within connector port 14 of connector header 12 of IMD 10 (Figs. 1 and 7), which is described herein in conjunction with Figure 1. Figure 8 further illustrates first lead 450 including a second, a third and a fourth insulated conductor 453, 454, and 455 coupling connector elements 463, 464, and 465 of connector terminal 460 to connector contacts 473, 474, and 475, respectively, included in port 470. According to embodiments of the present invention, port 470 is adapted to engage a connector terminal of a second lead, for example a connector terminal 420 of a second lead 400 illustrated in Figure 8, thereby supplementing second lead 400 with an additional high-voltage electrode, i.e. electrode 451, to create a more effective defibrillation vector, as previously described. In the exemplary system illustrated by Figure 8, second lead 400 includes a tip electrode 401 and a ring electrode 402, adapted for pacing and sensing, and a first high-voltage electrode 403 and a second high-voltage electrode 404; each electrode 401, 402, 403 and 404 are coupled to connector elements 421, 422, 423 and 424, respectively, of connector terminal 420 via insulated conductors 411, 412, 413 and 414, respectively. According to embodiments of the present invention, connector terminal 420 of second lead 400, like connector terminal 460 of first lead 450, is adapted to engage within connector port 14 of connector header 12 of IMD 10, however, when connector terminal 420 of second lead is engaged within port 470 of second lead, connector elements 421, 422, 423 and 424 are coupled to connector elements 465, 464, 463 and 462, respectively, which are then engaged by connector port 14 of IMD 10.

Although Figure 8 illustrates second lead 400 as a quadripolar lead including electrodes 401, 402, 403 and 404 wherein additional electrode 451 is joined in common with high-voltage electrode 404 via port 470, in alternate embodiments according to the present invention, a second lead may be a tripolar lead which does not include high-voltage electrode 404. In additional embodiments a second lead may not include low-voltage electrode 402; further combinations of electrodes understood by those skilled in the art are within the scope of the present invention.

Illustrative embodiments of systems incorporating means for connecting multiple high-voltage leads to an IMD having a single connection port have been described herein. While the present invention has been described in the context of specific embodiments, these embodiments are intended to be exemplary and are not intended to limit the scope, applicability, or configuration of the invention in any way. It should be understood that various changes may be made in the function and arrangement of elements described in exemplary embodiments without departing from the scope of the invention as set forth in the appended claims.

## Claims

1. A medical system, comprising:
a first lead (740) including a first electrode (758), a first insulated conductor (708), a second insulated conductor, a connector terminal (720), and an auxiliary connector port (734); the auxiliary connector port including a connector contact (744) and the lead connector terminal including a first connector element (728) coupled to the first electrode via the first conductor, a second connector element coupled only to the connector contact of the auxiliary port via the second conductor; a second lead (60) including an electrode (66) adapted for high-voltage therapy, an insulated conductor (714), and a connector terminal (62); the connector terminal of the second lead including a connector element (64) coupled to the electrode via the conductor; and an implantable medical device (10) including a connector port (14) including a first connector and a second connector; wherein, the auxiliary port of the first lead is adapted to engage the connector terminal of the second lead; and the connector port of the implantable medical device is adapted to engage the connector terminal of the first lead thereby coupling the first connector element of the first lead, via the first connector, and the second connector element of the first lead, via the second connector, to the implantable medical device.

2. The system of claim 1, wherein the first electrode is adapted for high-voltage therapy.

## Patentansprüche

1. Medizinisches System, mit:
einer ersten Leitung (740), die eine erste Elektrode (758), einen ersten isolierten Leiter (708), einen zweiten isolierten Leiter, einen Verbinderanschluss (720) und einen Hilfsverbinderport (734) aufweist; wobei der Hilfsverbinderport einen Verbinderkontakt (744) aufweist und wobei der Leitungsverbinderanschluss ein erstes Verbinderelement (728), das mit der ersten Elektrode über den ersten Leiter gekoppelt ist, ein zweites Verbinderelement, das nur mit dem Verbinderkontakt des Hilfsverbinderports über den zweiten Leiter gekoppelt ist; einer zweiten Leitung (60), die eine Elektrode (66), die für eine Hochspannungstherapie ausgelegt ist, einen isolierten Leiter (714) und einen Verbinderanschluss (62) aufweist; wobei der Verbinderanschluss der zweiten Leitung ein Verbinderelement (64) aufweist, das mit der Elektrode über den Leiter gekoppelt ist; und einer implantierbaren medizinischen Vorrichtung (10), die einen Verbinderport (14) aufweist, der einen ersten Verbinder und einen zweiten Verbinder enthält; wobei der Hilfsanschluss der ersten Leitung dazu ausgelegt ist, mit dem Verbinderanschluss der zweiten Leitung in Eingriff zu gelangen; und wobei der Verbinderanschluss der implantierbaren medizinischen Vorrichtung dazu ausgelegt ist, mit dem Verbinderanschluss der ersten Leitung in Eingriff zu gelangen, um **dadurch** das erste Verbinderelement der ersten Leitung über den ersten Verbinder und das zweite Verbinderelement der ersten Leitung über den zweiten Verbinder mit der implantierbaren medizinischen Vorrichtung zu koppeln.

2. System nach Anspruch 1, wobei die erste Elektrode für eine Hochspannungstherapie ausgelegt ist.

## Revendications

1. Système médical, comportant :
une première dérivation (740) incluant une première électrode (758), un premier conducteur isolé (708), un second conducteur isolé, une borne de connecteur (720), et un orifice de connecteur auxiliaire (734) ; l'orifice de connecteur auxiliaire incluant un contact de connecteur (744) et la borne de connecteur de dérivation incluant un premier élément de connecteur (728) couplé à la première électrode via le premier conducteur, un second élément de connecteur couplé uniquement au contact de connecteur de l'orifice auxiliaire via le second conducteur ; une seconde dérivation (60) incluant une électrode (66) adaptée en vue d'une thérapie haute tension, un conducteur isolé (714), et une borne de connecteur (62) ; la borne de connecteur de la seconde dérivation incluant un élément de connecteur (104) couplé à l'électrode via le conducteur ; et un dispositif médical implantable (10) comportant un orifice de connecteur (14) incluant un premier connecteur et un second connecteur ; dans lequel, l'orifice auxiliaire de la première dérivation est adapté pour mettre en prise la borne de connecteur de la seconde dérivation ; et l'orifice de connecteur du dispositif médical implantable est adapté pour mettre en prise la borne de connecteur de la première dérivation de manière à coupler le premier élément de connecteur de la première dérivation, via le premier connecteur, et le second élément de connecteur de la première dérivation, via le second connecteur, au dispositif médical implantable.

2. Système selon la revendication 1, dans lequel la première électrode est adaptée en vue d'une thérapie haute tension.
